Europäisches Patentamt

European Patent Office

Office européen des brevets

(11) Publication number: **0 046 338**
**B1**

## EUROPEAN PATENT SPECIFICATION

(45) Date of publication of patent specification: **10.04.85**

(51) Int. Cl.⁴: **A 61 F 2/14**

(21) Application number: **81303304.0**

(22) Date of filing: **17.07.81**

(54) Intraocular lens.

(30) Priority: **05.08.80 GB 8025426**
**11.05.81 GB 8114325**

(43) Date of publication of application:
**24.02.82 Bulletin 82/08**

(45) Publication of the grant of the patent:
**10.04.85 Bulletin 85/15**

(84) Designated Contracting States:
**AT BE CH DE FR IT LI LU NL SE**

(56) References cited:
**EP-A-0 024 126**
**DE-A-2 607 462**
**GB-A-2 046 099**
**US-A-4 092 743**
**US-A-4 163 609**

**APPLIED OPTICS, vol. 18, no. 11, June 1979,**
**pages 1842-1846; J.D. LYTLE et al.: "Wideband**
**optical transmission properties of seven**
**thermoplastics"**

(73) Proprietor: **Choyce, David Peter**
**9, Drake Road**
**Westcliff-on-Sea Essex SS0 8LR (GB)**

(72) Inventor: **Choyce, David Peter**
**9, Drake Road**
**Westcliff-on-Sea Essex SS0 8LR (GB)**

(74) Representative: **Messulam, Alec Moses et al**
**A. Messulam & Co. 24 Broadway**
**Leigh on Sea Essex SS9 1BN (GB)**

Courier Press, Leamington Spa, England.

## Description

The invention relates to an implant to be surgically inserted in any eye in order to correct defects in eyesight.

There have already been proposed artificial lenses for implantation in a human eye. Such implants have hitherto been intended not as corrective lenses but as substitutes for the natural lens in the eye. When an eye develops a cataract, the natural lens becomes fogged or opaque thereby impairing vision and when such a cataract is treated the lens is removed leaving the eye aphakic. It is possible to correct for aphakia using spectacles but because of the degree of correction required the thickness of the spectacles is substantial making them both cumbersome and unattractive.

For this reason, lenses have been designed for correction of aphakia in which the lens is inserted into a part of the eye either during the operation to remove the cataract or as a secondary operation. Such lenses fall into one of three categories namely anterior chamber implants, posterior chamber implants and iris plane implants, depending on the location of the lens in the eye. Such lenses are of fixed focal length and as the natural lens has been removed the eye is no longer capable of accommodation, that is to say the focal length cannot change to focus at different distances.

It is clear from what has already been said in relation to lenses previously inserted in an eye that these have been remedies adopted as a last resort for treating very serious eye conditions and such lenses could never be prescribed as an alternative to conventional spectacles for a person suffering from long sight, short sight or astigmatism.

Another form of implant which has been used in the past with some success has been the artificial cornea such as described in US Pat. No. 2,714,721, filed in January 23 1952, and generally resembling what is known as a kerato-prosthesis. These implants are a replacement for the natural cornea where the cornea has become opaque or fogged as many occur as a result of burns.

Because kerato-prostheses are a replacement for the damaged part of the cornea, after their insertion between the layers of the natural cornea a window is cut out of at least the damaged outer layer to permit the eye to see through the prosthesis which acts as a clear window in place of the natural cornea. If the inner layer of the cornea is undamaged then it is only necessary to trephine the outer layer of the cornea leaving the aqueous humour separated from the surrounding air by the inner layer of the cornea backed by the kerato-prosthesis. Should the inner layer of the cornea also be damaged then it too must be removed leaving the prosthesis as the only layer between the aqueous humour and the ambient air. It will thus be noted that the kerato-prosthesis is always in contact with the air and is held in place by the portions of the natural cornea which remain only around its periphery.

It can once again be seen that such an implant would on no account be considered an alternative to the use of spectacles or contact lenses.

It is known to resort to surgery in order to correct for defects in eyesight. In particular, a surgical technique has been developed (radial keratotomy) in which radial incisions are made in the cornea with the intention that the cornea should heal with a different curvature from its original, thereby correcting the eyesight defect. The number of incisions and their dimensions are varied to allow for different degrees of correction.

Such surgery can never have a fully predictable outcome and furthermore any non-spherical flattening of the cornea on healing results in an eyesight defect which cannot be corrected by the use of spectacles or contact lenses.

It can therefore be seen that despite the known problems and inconvenience caused to wearers of spectacles and contact lenses, no alternative satisfactory proposal has yet been made and it is an object of the present invention to mitigate the foregoing problems.

In accordance with the present invention, there is provided a lens for insertion between the layers of the cornea in order to correct defects in eyesight, characterised by being formed of a polysulfone plastics material.

Because the lens is made of a polysulfone, the lens has a high refractive index relative to that of the cornea, thereby enabling a relatively thin lens to achieve correction of several dioptres.

It will be noted that this implant differs from that of the earlier mentioned kerato-prosthesis in as much as after its insertion the inlay is entirely embedded in the cornea and is not exposed to the atmosphere nor to the aqueous humour. By contrast, in a kerato-prosthesis, the implant is not surrounded on both sides by layers of cornea.

A significant advantage is gained by the use of polysulfone in the making of the inlay because the lens can be sterilised prior to insertion by steam autoclaving. This considerably reduces the cost of packaging the inlays which need not be packaged in individual sterile containers.

It has been found that because of the high refractive index of polysulfones and in particular of Udel (Registered Trade Mark) as sold by the Union Carbide Corporation, it is possible to correct up to +10 dioptres at spectacle distance with a lens of which the maximum thickness is only 0.4 mm and that one can correct −10 dioptres with a differently shaped lens with a thickness of only 0.1 mm at its centre.

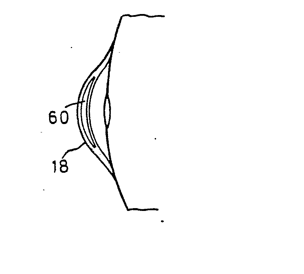

The invention will now be described further, by way of example, with reference to the accompanying drawing which shows a section through an eye fitted with an intracorneal inlay.

In the accompanying drawing, there is shown an intracorneal inlay 60 which has been inserted just in front of the inner lining membrane of the cornea 18 known as Descemet's membrane.

Experimental attempts at the insertion of such an inlay were made several years ago using an inlay made of glass, but these were not well documented because of their lack of success.

Though the technique for the separation of the layers of cornea to insert an implant is known intracorneal inlays have not been successful because of the difficulty in selection of an appropriate material. The glass inlays which were experimented with in the past had no flexibility and tended to fracture. Furthermore, because of the relatively lower refractive index of the glasses used, the lenses were of considerable thickness and this in turn caused clinical problems.

The present invention proposes an inlay 60, to be inserted between the layers of the cornea 18, which is made of a polysulfone plastics material, the preferred material being Udel (Registered Trade Mark). The latter material has a high refractive index enabling inlays of substantial power to be made of small thickness.

It has been found that by resorting to the use of polysulfone material it is possible to use the intracorneal inlay to correct or alleviate myopia, hypermetropia and astigmatism. Insertion of the lens does not require puncturing of the anterior chamber and is readily within the capability of an experienced corneal surgeon.

All lenses which are to be implanted in the eye must be sterile before their insertion and the problem with sterilisation is one which is well known from other intra-ocular lenses such as anterior chamber implants.

Polysulfone plastics not only have the advantage of being clinically inert but have high temperature properties which permit them to be sterilised by steam autoclaving.

The preferred material, Udel, has a refractive index in the region of 1.65 which is higher than the refractive index of the cornea and it is possible to correct +10 dioptres of hypermetropia at spectacle distance with an intracorneal lens made of Udel of which the maximum thickness is only 0.4 mm. In order to provide +10 dioptres, a positive lens may be formed with a diameter of 6 mm, a concave surface with a radius of curvature of 6.85 mm and a convex surface with a radius of curvature of 5.546 mm. If the thickness of the edges is 0.21 mm then the thickness of the centre will amount to 0.4 mm.

Similarly, one can correct up to 10 dioptres of myopia with a differently shaped lens which is only 0.1 mm thick at its centre. To form a negative lens of a power of −10 dioptres, a lens of diameter of 6 mm may have a convex surface with a registered curvature of 8.025 mm and a concave surface with a radius of curvature of 6.85 mm. If the thickness of the edges is 0.2 mm, the central thickness is 0.1 mm.

The dimensions of these inlays are not too different from contact lens parameters and they can therefore be readily manufactured by the use of available techniques. The curvatures and thicknesses chosen for the lenses described above are designed to fit in the stroma as snugly as possible.

It is believed that polysulfone plastics material other than Udel have important properties for the present application, namely a high refractive index, chemical inertness and the ability to withstand autoclaving. Udel offers the further advantage of flexibility which reduces the risk of fracture.

## Claims

1. A lens for insertion between the layers of the cornea in order to correct defects in eyesight, characterised by being formed of a polysulfone plastics material.

2. A lens as claimed in Claim 1, wherein the polysulfone plastics materials is Udel (Registered Trade Mark).

3. A lens as claimed in Claim 1 or Claim 2, in which the lens is sterilised by steam autoclaving.

## Patentansprüche

1. Linse zum Einsetzen zwischen den Schichten der Kornea zur Korrektur von Sehfehlern, dadurch gekennzeichnet, dass sie aus einem Polysulfonkunststoff gebildet ist.

2. Linse nach Anspruch 1, worin als Polysulfonkunststoff Udel (eingetragenes Warenzeichen) vorliegt.

3. Linse nach Anspruch 1 oder 2, sterilisiert mittels Dampf im Autoklaven.

## Revendications

1. Lentille pour l'insertion entre les couches de la cornée pour corriger des défauts de la vue, caractérisée en ce qu'elle est formée d'une matière plastique de polysufone.

2. Lentille suivant la revendication 1, dans laquelle la matière plastique de polysulfone est l'Udel (marque déposée).

3. Lentille suivant la revendication 1 ou la revendication 2, caractérisée en ce que la lentille est stérilisée par autoclavage à la vapeur.